Europäisches Patentamt

European Patent Office

Office européen des brevets

(19)

(11)     **EP 1 647 911 A2**

(12)                    **EUROPEAN PATENT APPLICATION**

(43) Date of publication:
      **19.04.2006  Bulletin 2006/16**

(51) Int Cl.:
      *G06F 19/00* (2006.01)

(21) Application number: 05256067.9

(22) Date of filing: **29.09.2005**

(84) Designated Contracting States:
      **AT BE BG CH CY CZ DE DK EE ES FI FR GB GR
      HU IE IS IT LI LT LU LV MC NL PL PT RO SE SI
      SK TR**
      Designated Extension States:
      **AL BA HR MK YU**

(30) Priority: **12.10.2004  US 964524**

(71) Applicant: **Agilent Technologies, Inc.**
      **Palo Alto, CA 94306 (US)**

(72) Inventor: **Kincaid, Robert**
      **Half Moon Bay, California 94019 (US)**

(74) Representative: **Tollett, Ian et al**
      **Williams Powell**
      **Morley House**
      **26-30 Holborn Viaduct**
      **London EC1A 2BP (GB)**

(54)     **Systems and methods for statistically analyzing apparent CGH Data Anomalies**

(57)     Methods, systems and computer readable media for statistically analyzing apparent anomalies in CGH data, wherein the CGH data is ordered corresponding to locations of matter on chromosomes from which the CGH data was derived. A set of CGH ratio values is considered and Z-score values (106) are computed for each CGH ratio value. The Z-score values are classified (108) based upon a predetermined cutoff value. The number of Z-scores that are greater than the predetermined cutoff value are counted, the number of Z-scores that are less than a negative of the predetermined cutoff value are counted, and the total number of Z-scores are counted. A subset of the set of CGH ratios are considered, being defined by a window of predetermined size. A secondary Z-score (116) is computed to measure the significance of at least one of overabundance and underabundance of at least one of significant positive deviations and significant negative deviations in the subset.

Input CGH ratio value ∿102

Convert CGH ratios to log ratios ∿104

Convert Z-score for each log ratio value ∿106

Classify Z-score values ∿108

Determine R, R' and N ∿110

For each window w, determine s, r' and n ∿112

Calculate exact Z-scores ∿114

Plot exact Z-scores ∿116

Filter display using cutoff value Z c' ∿118

*FIG. 1*

**Description**

The present invention relates to a system and methods for statistically analyzing apparent anomalies in comparative genomic hybridization (CGH) data.

Alterations in DNA copy number are characteristic of many cancer types and are thought to drive some cancer pathogenesis processes. These alterations include large chromosomal gains and/or losses, as well as smaller scale amplifications and/or deletions.

The mapping of common genomic aberrations has been a useful approach to discovering cancer-related genes. Genomic instability may trigger the over-expression or activation of oncogenes and the silencing of tumor suppressors and DNA repair genes. Local fluorescence in-situ hybridization-based techniques were used early on for measurement of alterations in DNA copy number.

A genome-wide measurement technique referred to as Comparative Genomic Hybridization (CGH) is currently used for identification of chromosomal alterations in cancer, e.g., see Balsara et al., "Chromosomal imbalances in human lung cancer", *Oncogene,* 21(45):6877-83, 2002; and Mertens et al., "Chromosomal imbalance maps of malignant solid tumors: a cytogenetic survey of 3185 neoplasms", *Cancer Research,* 57(13):2765-80, 1997. Using CGH, differentially labeled tumor and normal DNA are co-hybridized to normal metaphases. Ratios between the tumor and normal labels enable the detection of chromosomal amplifications and deletions of regions that may include oncogenes and tumor suppressive genes. This method has a limited resolution however, of only about 10-20 Mbp (mega base pairs). This amount of resolution provided is insufficient to enable a determination of the borders of the chromosomal changes or to identify changes in copy numbers of single genes and small genomic regions.

A more advanced measurement technique referred to as array CGH (aCGH) enables the determination of changes in DNA copy number of relatively small chromosomal regions. Using aCGH, tumor and normal DNA are co-hybridized to a microarray of thousands of genomic clones of BAC, cDNA or oligonucleotide probes, e.g., see Pollack et al., "Genome-wide analysis of dna copy number changes using cdna microarrays", *Nature Genetics,* 23(1):41-6, 1999; Pinkel et al., "High resolution analysis of dna copy number variation using comparative genomic hybridization to microarrays", *Nature Genetics,* 20(2):207-211, 1998; and Hedenfalk et al., "Molecular classification of familial non-brca1/brca2 breast cancer", *PNAS.* By using oligonucleotide arrays, the resolution provided can, in theory, be finer than that necessary to identify single genes.

An ongoing problem with aCGH data, is that it is currently very noisy and thus it is difficult to determine whether anomalous data values are the result of a real anomaly (amplification or deletion) occurring in the test subject matter, or whether the anomaly is largely the result of noise and that a real anomaly is not present. Current approaches to manipulating or analyzing aCGH data have been taken in an effort to separate noise from actual occurrences of anomalies. One such approach, discussed in Ben-Dor et al., "Analysis of Array Based Comparative Genomic Hybridization Data - Theory and Validation", is based on computing hypergeometric p-values from the data. In some cases, this approach uses dynamic programming to further refine the result. While this method provides highly rigorous results, the computations performed are relatively intensive, and are not easily supported in a dynamic, interactive display.

Crawley et al., in "Identification of frequent cytogenetic aberrations in hepatocellular carcinoma using gene-expression microarray data", endeavors to identify cytogenic aberrations by considering gene-expression microarray data, thus avoiding the issues with interpreting aCGH data. Gene expression values are analyzed and a sign test is applied to identity whether a significant upward of downward bias is present in the expression values. This is not a statistically based metric. Approximations to actual z-scores are then generated based on the results of the sign test.

There remains a current need for fast and universally usable techniques for analyzing aCGH data, since current arrays typically produce very noisy results and care must be taken not to interpret dramatic but statistically irrelevant deviations as being biologically relevant.

According to a first aspect of the present invention there is provided a system for statistically analyzing apparent anomalies in CGH data as claimed in claim 1.

According to a second aspect of the present invention there is provided a method for statistically analyzing apparent anomalies in CGH data as claimed in claim 23.

According to a third aspect of the present invention there is provided a method for statistically analyzing apparent anomalies in CGH data as claimed in claim 24.

Methods, systems and computer readable media are provided for statistically analyzing apparent anomalies in CGH data, wherein the CGH data is ordered corresponding to locations of matter on chromosomes from which the CGH data was derived. A set of CGH ratio values are considered, and a Z-normalized value for each CGH ratio value is computed. The Z-normalized values are classified, based upon a predetermined cutoff value, and the number of Z-normalized values that are greater than the predetermined cutoff value are counted, the number of Z-normalized values that are less than a negative of the predetermined cutoff value are counted, and the total number of Z-normalized values are counted. A subset of the set of CGH ratios is considered, the subset being defined by a window of predetermined size. A Z-score is then computed to measure the significance of at least one of overabundance and underabundance of at least one of significant positive deviations and significant negative deviations in the subset.

Methods, systems and computer readable media are provided for statistically analyzing apparent anomalies in CGH data, wherein the CGH data includes a set of CGH ratio values ordered corresponding to locations of matter on chromosomes from which the CGH data was derived, a Z-normalized value has been computed for each CGH ratio value, the Z-normalized values have been classified based upon a predetermined cutoff value, and the number of Z-normalized values that are greater than the predetermined cutoff value, the number of Z-normalized values that are less than a negative of the predetermined cutoff value, and the total number of Z-normalized values have been counted. A subset of the set of CGH ratios is considered, as defined by a window of predetermined size. A Z-score is then computed to measure the significance of at least one of overabundance and underabundance of at least one of significant positive deviations and significant negative deviations in the subset.

Methods, systems and computer readable media are provided for statistically analyzing apparent anomalies in CGH data, wherein the CGH data includes a set of CGH ratio values ordered corresponding to locations of matter on chromosomes from which the CGH data was derived. A subset of the set of CGH ratios are defined by a window of predetermined size and considered. A Z-test between the data within the window and statistics derived from the set of CGH ratios is computed according to the following:

$$Z = \sqrt{n}\,\frac{\bar{X} - \mu}{\sigma}$$

where
$Z$ is the calculated value of the Z-test;
$n$ is the number of values within said window;
$\bar{X}$ is the mean of the values within said window;
$\mu$ is the mean of the values in said set; and
$\sigma$ is the standard deviation of the values in said set. A moving average of the subset of values in the window is also calculated.

Methods, systems and computer readable media are provided for statistically analyzing apparent anomalies in CGH data, wherein the CGH data includes a set of CGH ratio values ordered corresponding to locations of matter on chromosomes from which the CGH data was derived. A subset of the set of CGH ratios, defined by a window of predetermined size, is considered. A t-test between the data within the window and statistics derived from the set of CGH ratios is computed according to the following:

$$t = \sqrt{n}\,\frac{\bar{X} - \mu}{s}$$

where
$t$ is the calculated value of the t-test;
$n$ is the number of values within said window;
$\bar{X}$ is the mean of the values within said window;
$\mu$ is the mean of the values in said set; and
$s$ is the standard deviation of the values within said window. A moving average of the subset of values in the window is also calculated.

A user interface, methods and computer readable media are provided for displaying various graphical representations of CGH data values and apparent anomalies in the CGH data values via means for displaying a chromosome map and means for plotting statistical scores of aberration characterizing the CGH data values adjacent the chromosome map, in areas corresponding to locations of matter from which the CGH data values were derived.

A user interface, methods and computer readable media are provided for displaying various graphical representations of CGH data values and apparent anomalies in the CGH data values, wherein the CGH data includes a set of CGH ratio values ordered corresponding to locations of matter on chromosomes from which the CGH data was derived, a Z-normalized value has been computed for each CGH ratio value, the Z-normalized values have been classified based upon a predetermined cutoff value, and the number of Z-normalized values that are greater than the predetermined cutoff value, the number of Z-normalized values that are less than a negative of the predetermined cutoff value, and the total number of Z-normalized values have been counted; and wherein Z-scores have been computed for subsets of the set of CGH ratios incrementally defined by a window of predetermined size, to measure the significance of at least one of overabundance and underabundance of at least one of significant positive deviations and significant negative deviations in the subsets, wherein the user interface includes means for displaying a chromosome map; and means for plotting plots the Z-scores adjacent the chromosome map, in areas corresponding to the locations of the matter from which the CGH data values in the windows were derived, for each respective Z-score.

A user interface, methods and computer readable media are provided for displaying various graphical representations of CGH data values and apparent anomalies in the CGH data values, wherein the CGH data includes a set of CGH ratio values ordered corresponding to locations of matter on chromosomes from which the CGH data was derived, where the user interface includes means for displaying a chromosome map; means for displaying at least one of moving average values calculated from the CGH data values, and the CGH data values; and means for overlaying statistical scores characterizing apparent anomalies in the CGH data values.

These and other advantages and features of the invention will become apparent to those persons skilled in the art upon reading the details of the systems, user interfaces, methods and computer readable media as more fully described below.

Preferred embodiments of the invention will now be described by way of example only and with reference to the drawings in which:

Fig. 1 shows a flow chart of processing steps that may be carried out with the present system to statistically analyze apparent anomalies in CGH data.
Fig. 2 shows an exemplary display on which Z-scores for one experiment have been plotted, relative to moving averages.
Fig. 3 is a display similar to that shown in Fig. 2, but where data for multiple experiments has been plotted.
Fig. 4 is another view of a display, similar to Figs. 2 and 3, and wherein, additionally, a scatter plot of data points is displayed.
Fig. 5 is a zoomed view to show plotted data in more detail
Fig. 6 is another zoomed view, similar to Fig. 5, but where moving average data has not been plotted or displayed.
Fig.7 shows a portion of the data outputted and displayed by a text reporter provided by the present invention.
Fig. 8 shows an interface provided to a user for selecting Z-scores to determine which experimental data to plot.
Fig. 9 illustrates a typical computer system in accordance with an embodiment of the present invention.

Before the present methods, systems and computer readable media described, it is to be understood that this invention is not limited to particular examples described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a data value" includes a plurality of such data values and reference to "the array" includes reference to one or more arrays and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

A "microarray", "bioarray" or "array", unless a contrary intention appears, includes any one-, two-or three-dimensional arrangement of addressable regions bearing a particular chemical moiety or moieties associated with that region. A microarray is "addressable" in that it has multiple regions of moieties such that a region at a particular predetermined location on the microarray will detect a particular target or class of targets (although a feature may incidentally detect non-targets of that feature). Array features are typically, but need not be, separated by intervening spaces. In the case of an array, the "target" will be referenced as a moiety in a mobile phase, to be detected by probes, which are bound to the substrate at the various regions. However, either of the "target" or "target probes" may be the one, which is to be evaluated by the other.

Methods to fabricate arrays are described in detail in US Patent 6,242,266; 6,232,072; 6,180,351; 6,171,797 and 6,323,043. As already mentioned, these references are incorporated herein by reference. Other drop deposition methods can be used for fabrication, as previously described herein. Also, instead of drop deposition methods, photolithographic array fabrication methods may be used. Interfeature areas need not be present particularly when the arrays are made by photolithographic methods as described in those patents.

Following receipt by a user, an array will typically be exposed to a sample and then read. Reading of an array may be accomplished by illuminating the array and reading the location and intensity of resulting fluorescence at multiple regions on each feature of the array. For example, a scanner may be used for this purpose is the AGILENT MICROARRAY SCANNER manufactured by Agilent Technologies, Palo, Alto, CA or other similar scanner. Other suitable apparatus and methods are described in US Patents 6,518,556; 6,486,457; 6,406,849; 6,371,370; 6,355,921; 6,320,196; 6,251,685 and 6,222,664. However, arrays may be read by any other methods or apparatus than the foregoing, other reading methods including other optical techniques or electrical techniques (where each feature is provided with an electrode to detect bonding at that feature in a manner disclosed in US Patents 6,251,685, 6,221,583 and elsewhere).

The acronym "CGH" refers to Comparative Genomic Hybridization.

The acronym "aCGH" refers to microarray-based CGH.

The term "aCGH array " refers to a microarray used to perform an aCGH experiment. Typically, an aCGH array or aCGH microarray is designed specifically for CGH measurements, in which case probes are designed to hybridize with genomic DNA. However, in some cases a standard expression array can be used, since the DNA probes designed to measure RNA will also be complementary to the genomic DNA coding for those transcripts.

When one item is indicated as being "remote" from another, this is referenced that the two items are at least in different buildings, and may be at least one mile, ten miles, or at least one hundred miles apart.

"Communicating" information references transmitting the data representing that information as electrical signals over a suitable communication channel (for example, a private or public network).

"Forwarding" an item refers to any means of getting that item from one location to the next, whether by physically transporting that item or otherwise (where that is possible) and includes, at least in the case of data, physically transporting a medium carrying the data or communicating the data.

A "processor" references any hardware and/or software combination which will perform the functions required of it. For example, any processor herein may be a programmable digital microprocessor such as available in the form of a mainframe, server, or personal computer. Where the processor is programmable, suitable programming can be communicated from a remote location to the processor, or previously saved in a computer program product. For example, a magnetic or optical disk may carry the programming, and can be read by a suitable disk reader communicating with each processor at its corresponding station.

Reference to a singular item, includes the possibility that there are plural of the same items present.

"May" means optionally.

Methods recited herein may be carried out in any order of the recited events which is logically possible, as well as the recited order of events.

All patents and other references cited in this application, are incorporated into this application by reference except insofar as they may conflict with those of the present application (in which case the present application prevails).

The present invention provides methods, systems and computer readable media for determining whether apparent anomalous values from CGH data such as aCGH data, for example, are statistically valid, or are, instead within the distribution of noise associated with the data.

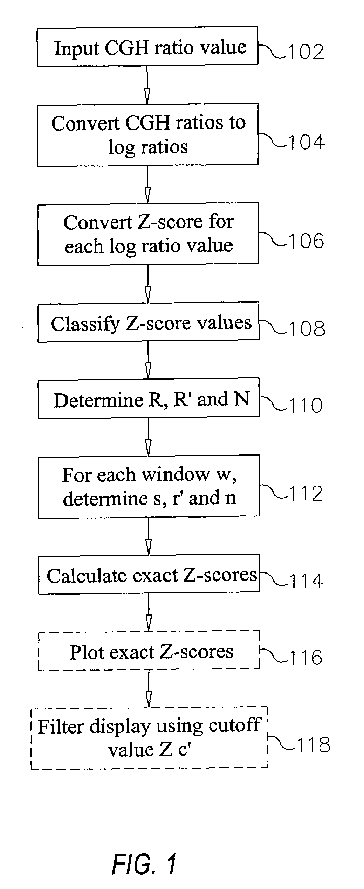

Referring now to Fig. 1, a flow chart of processing steps that may be carried out with the present system to statistically analyze apparent anomalies in CGH data is shown. At event 102, a dataset of CGH ratio values, such as read from an aCGH array, for example, are inputted. The CGH ratio values are next converted to log ratio values at step 104. Each log ratio value in the dataset is then z-normalized, by computing a Z-normalized value for each log ratio value $x$, as follows:

$$Z(x) = \frac{x - \mu}{\sigma} \qquad (1)$$

where

$x$ is the log of a measured CGH ratio;

$\mu$ is the mean of the log ratio values; and

$\sigma$ is the standard deviation of the population of the log ratio values.

The values for $\mu$ and $\sigma$ may be calculated based on a population taken from a single chromosome, an entire array, or over an entire collection of experiments. Alternatively, the values for $\mu$ and $\sigma$ may be derived from specific calibration experiments designed specifically to characterize these statistical parameters. The choice among which population to use may depend upon the experimental context. For example, if all arrays from which CGH ratios were taken were of identical design and were processed with similar protocols, then averaging over all arrays may give the more accurate estimate of values for $\mu$ and $\sigma$. However, if there are different array types in use and/or protocols or conditions where some arrays have broader distributions of values than others, then more accurate estimates of values for $\mu$ and $\sigma$ may be obtained by calculating the values on a per array basis, or per array class basis. Further, additional considerations/corrections may need to be made to account for X and Y chromosomes (gender), since these values may also potentially skew $\mu$ and $\sigma$ erroneously. Thus, typically values for X and Y chromosome are not considered for calibrating the mean and standard deviation values. By not using values from the X and Y chromosomes, gender differences among the data being considered will not affect the computation of the mean and standard deviation values. However, data from the X and Y chromosomes, may be included for calculation of the mean and standard deviation if compensation is made for different numbers generated from these chromosomes between male and female sources. For simplicity, X and Y chromosome values are not considered, as noted, so that the user does not need to track the gender for the data being considered.

At event 108, the Z-normalized values are classified as to whether they are significantly above or below the mean $\mu$, or neither, by comparing the values with a predetermined cutoff value $Z_c$ (e.g., $Z_c = 3$). The predetermined cutoff value is not limited to 3, however, and may be set by a user, i.e., a user-specified value. The system then determines values that are greater than $Z_c$ or less than negative $Z_c$ to be significantly above or below the mean, respectively.

The number of entries in three classes are then determined at event 110, based upon the results of the classification in event 108, as follows:

R = the number of entries(values) greater than $Z_c$,

R' = the number of entries(values) less than $-Z_c$, and

N = the total number of measurements (values).

The Z-normalized values (i.e., $Z(x)$) and counts for R, R' and N may be stored for subsequent calculations described below. Further, the scores calculated in event 106 may be reused for subsequent processing, should a user decide to change the value of $Z_c$ and then re-compute events 108 and 110.

Ideally, the global statistics for $\mu$ and $\sigma$ may be based on samples that contain no genetic anomalies, such that $\mu$ and $\sigma$ represent the distribution of a non-diseased sample. These global statistics may be calculated from all arrays available to the user that have no copy number anomalies, or from a user-defined set of calibration arrays, for example. However, a simplifying approximation may be used to take the statistics for an entire set of arrays, with the expectation that any genetic anomalies present in the entire set will provide only a small perturbation to $\mu$ and $\sigma$, when averaged over all arrays, which in turn are averaged over all chromosomes (except X and Y). Since only some chromosomes will typically show anomalous behavior in an entire set of arrays, the expectation as to the contribution of amplifications and deletions to the averaging statistics is expected to be small compared to the overall global behavior.

A common statistic that is currently calculated with regard to aCGH data is the moving average. While computing a moving average, log ratios are averaged over a small subset of points. A moving average "window" is passed over a set of data values to define a subset of the data values from which a moving average is calculated, for each position of the window. The moving average window may simply identify some predetermined number of adjacent measurements, or it may be over a positional window, such as over a megabase, for example. For each of these windows, there are n entries.

The present system employs a window w to analyze the over- or under- abundance of log ratios that significantly deviate from the mean calculated in event 106 and which lie within the window $w$. Moving averages may optionally be calculated at the same time that this processing is occurring, based on the same window $w$. For each position of the window $w$, counts similar to those computed in event 110 are made (event 112), only this time the counts are only for the subset identified by window $w$, as follows:

r = the number of entries(values) in $w$ greater than $Z_c$,

r' = the number of entries(values) in $w$ less than $-Z_c$,

n = the total number of measurements (values) in $w$,

R = the number of entries(values) greater than $Z_c$ in the full data set,

R' = the number of entries(values) less than $-Z_c$ in the full data set, and

N = the total number of measurements.

From these counts, a Z-score may be calculated (event 114) to measure the significance of the over/under-abundance in *w* of significant positive deviations (i.e., putative amplifications) as follows:

$$Z(w) = \frac{(r - n\frac{R}{N})}{\sqrt{n(\frac{R}{N})(1 - \frac{R}{N})(1 - \frac{n-1}{N-1})}} \qquad (2)$$

Similarly, a Z-score to measure the significance of the over/under-abundance in *w* of significant negative deviations (i.e., putative deletions) may be calculated as follows:

$$Z(w) = \frac{(r' - n\frac{R'}{N})}{\sqrt{n(\frac{R'}{N})(1 - \frac{R'}{N})(1 - \frac{n-1}{N-1})}} \qquad (3)$$

The scores calculated from equations (2) and (3) may be plotted (event 116) analogously to the way the moving averages are plotted, and such plots then indicate statistically significant groups of probes that appear to deviate for the typical distribution of values of the given experiments. Thus, the plots from the values calculated by equations (2) and (3) may be used as a predictive tool to identify potential amplification or deletion events in CGH studies. A second cutoff value or Z-score cutoff value, $Z_c'$ may be used (event 118) to eliminate from the display of Z-score plots those areas with statistically unimportant changes. Either or both $Z_c$ and $Z_c'$ scores may be changed or adjusted by the user, if desired, as appropriate for the user's visual analysis of the resultant plots. Further, the user may also specify the window size *w*. Thus, the user may specify some reasonable window size (e.g., based on how dense the coverage of the array is) and a value for $Z_c$ based on how stringent the user desires the computations to be. For example, a relatively narrow window size (e.g., .5 Mb) and a high $Z_c$ (e.g., $Z_c$ = 4) may be chosen to give few statistical anomalies. However the statistical anomalies identified will have very high confidence that they are true positive anomalies. Alternatively, the parameters may be relaxed to identify greater numbers of anomalies, but with less confidence that all are true aberrations. As noted above, these computations can be readily performed in parallel with moving average computations, or may be carried out independently of any other calculations.

The user's choice for a window size and $Z_c$ value may be determined somewhat intuitively, either by playing with the parameters and seeing the resultant visualizations, or by thinking about what these parameters mean given the specifics of the data being considered. As noted, the $Z_c$ value can be whatever the user desires it to be, i.e., as to what the user determines to be statistically significant. Typically the value will be about $Z_c$ = 2, meaning that any points that are two standard deviations above the mean would be considered significant deviations or anomalies, although this can be varied. The size of the window chosen should be sufficient to include enough points in the window sample to give useful measurements. Typically around five to ten data points per window is sufficient. However, the Z-scoring algorithm will indicate if any windows are statistically relevant, so the user can manually experiment with various values and choose on that the user considers to best reflect the types of anomalies that the user is trying to observe. Short stretches of narrow amplifications or deletions will require a very narrow window size for detection, while amplifications of entire cytobands or chromosome arms may require a larger window size. On the other hand, setting a window size to capture one thousand points per sample is probably too large a window size in most instances. The scores discussed herein can be computed rapidly, and can be carried out a part of an interactive process. Further, since Z-scores have an easily understood interpretation as standard units of deviation from the mean, the present solution enables users/analysts to intuitively modify cutoff values, and/or moving average window sizes to adjust the calculations to their preferences. Results of such modifications can be viewed in a few seconds and are therefore useful as part of an overall exploratory analysis.

In addition, or alternative to the two-stage Z-scoring procedure discussed above, the system may calculate a Z-test or t-test between the statistics of the window w and the global mean and standard deviation values μ and σ (such as were calculated at event 106, for example). Like the previous procedure, this procedure may be carried out in parallel with moving average computations, or may be carried out independently, or along with the two-stage Z-scoring procedure. A one-sample Z-test may be formulated as:

$$Z = \sqrt{n} \frac{\bar{X} - \mu}{\sigma} \tag{4}$$

where

$n$ = the number of data points (values) in window $w$,

$\bar{X}$ = the means of the data points within window $w$, and

$\mu$ and $\sigma$ are the mean and the standard deviation of the entire population, i.e., the entire dataset over which window $w$ is being positioned, move by move. The global mean $\mu$ and global standard deviation $\sigma$ are assumed to be normal. If $\sigma$ is not known, then the standard deviation of the sample (i.e., standard deviation based only on values within window $w$ can be used, in which case, the procedure becomes a t-test, rather than a Z-test. Either way, this procedure offers an even simpler and faster computation of statistical scoring than the two-step Z-scoring procedure. However, since assumptions about normal distributions are made, these procedures can potentially be less accurate than the two-step Z-scoring procedures.

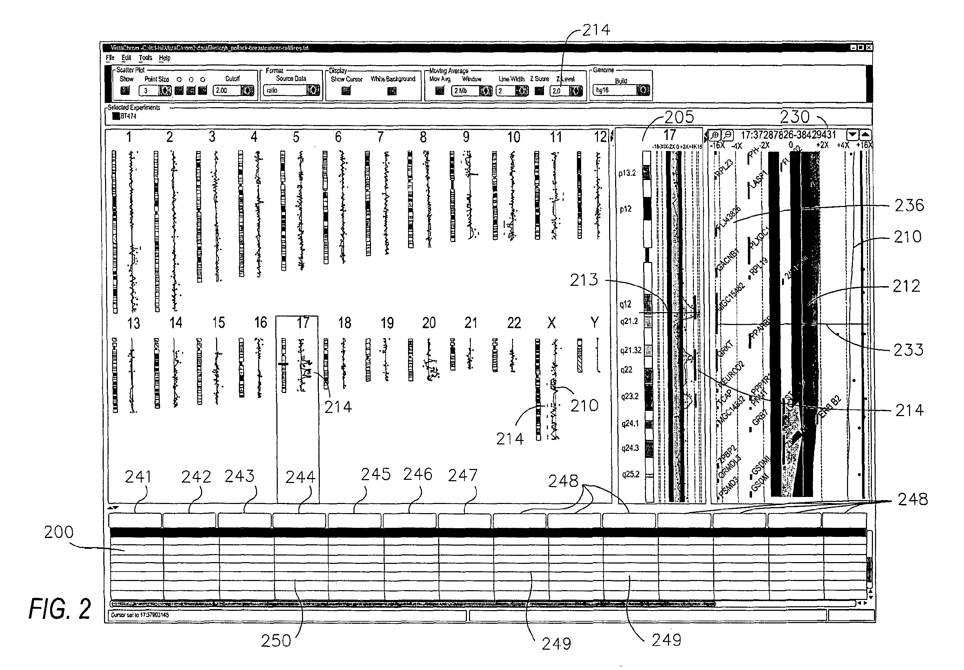

Once the final Z-scores have been computed, by whichever method, the Z-scores can be plotted as a line graph similar to the way in which a moving average is plotted. Fig. 2 shows an exemplary display 200 on which Z-scores 212 for one experiment have been plotted, relative to moving averages 210, for the sake of simplifying the drawing as much as possible. Of course, the present system can plot Z-scores, as well as moving averages for multiple experiments, as is often the case.

In the example shown, moving averages 210 and Z-scores 212 have been plotted relative to the selected chromosome (in this example, chromosome 17, shown outlined or selected 202 in the global map containing the unzoomed views of each chromosome), where the selected chromososme is shown in the zoomed view 205. The Z-scores plot 212 may be color-filled to the origin to make it appear more like a histogram, for easier visual distinction between it and a moving average plot 210. Additionally, when more than one experiment is plotted, the color-filled Z-scores plots may be alpha-blended for transparency, so that when the plots overlap, this minimizes obscuring data and allows detection of the overlaps. For two or three simultaneous plots, it is possible to distinguish the various possible intersections based on the color blending of the overlapping, differently colored plots. In the example shown, the Z-score plot 212 is reduced by a factor of ten, thus allowing the user to read off the actual underlying value by interpolating the location of the graph scale (i.e., $\pm 2$, $\pm 4$, etc.) and then multiplying this value by ten. The graph scale 215 may be read directly for the values of the moving average.

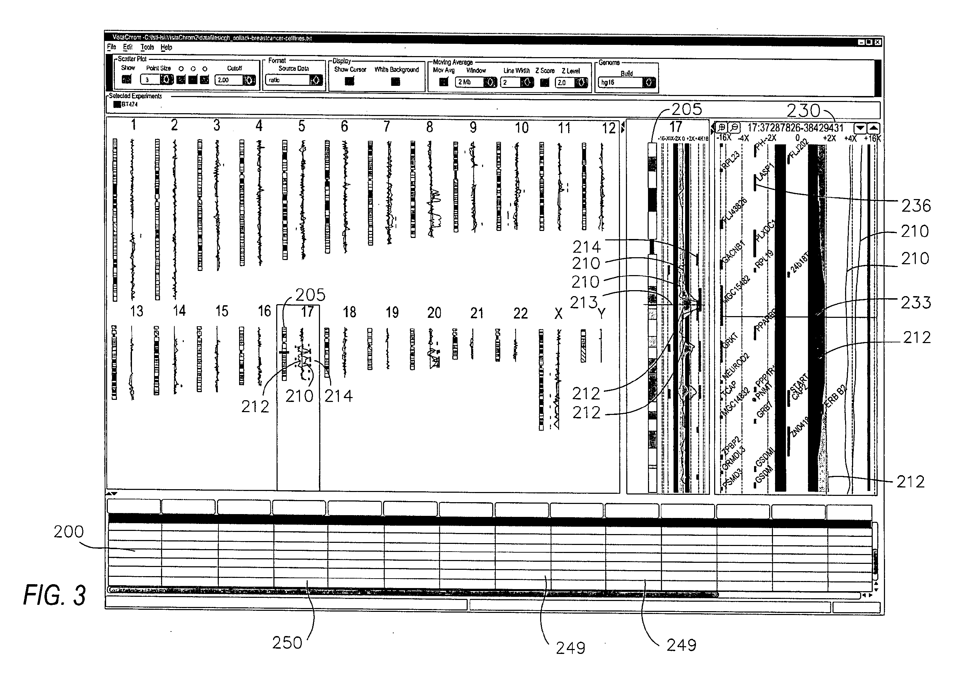

A detailed description of the chromosomal mapping and zooming features is contained in Application Serial No. 10/817,244, which was incorporated by reference above. Area 204 of display 200 displays annotations for the experimental data, e.g., "Unigene ID" 241, "Chromosome No." 242, "Start (hg16)" 243 "Stop (hg16)" 244 Name (hg16) 245, CLID 246 and Name 247 in this example, although the annotations that are displayed may vary. Also, the entries 250 in the rows beneath each of these headers are omitted, since they would be too small to meet drawing requirements. Columns 248 contain the actual experimental data values 249 (not shown, since numerals and text would be too small to meet drawing requirements) taken from the various experimental arrays. When an array is selected for display (e.g., experiment "BT474" has been selected in the example shown), a color may be assigned to distinguish the data for that experiment on the display. This is particularly useful when data for multiple experiments is being displayed, such as is shown in Fig. 3, for example.

Box 218 displays the user specified Z-value or Z-level (i.e., $Z_c$) that was used for the classification stage described above. This enables the user to input a user-specified cutoff value for classifying the Z-normalized values as described above. This value can be changed to process the same data according to different cutoff values, wherein the user can visually analyze the displays from each run with a different cutoff value to determine which value is most appropriate to the data being studied and for the user's current purposes.

Side bars 214 are plotted adjacent the Z-scores that are considered to be significant. In the example shown, only Z-scores greater than zero are plotted. Scores corresponding to putative amplifications are plotted to the right of zero and scores corresponding to putative deletions are plotted to the left of zero. In instances where more than one experiment is plotted such that there are multiple Z-score plots 212 (and optionally, multiple moving average plots 210), such as shown in Fig. 3, for example, a separate column is used for side bars relative to each experiment. Additionally, the plots 210, 212 may be color coded to the experiment, with each experiment being displayed appearing next to a color key. The sidebars 214 may then be color coded according to the same scheme. Sidebars 214 are also plotted against all of the chromosome maps in the global view for which there is data that meets the requirements for displaying a side bar. Typically, moving average plots 210 and Z-score plots 212 are not included adjacent the smaller chromosomes in the global view, because they become difficult to read, although they may optionally be displayed in this way, as shown in Figs. 2-3 for example.. Such an option may be adopted, for example, when there is a relatively simple display, such as when only one experiment is being displayed.

A zoomed view of the display of the moving average plots 210, Z-scores plots 212 and side bars 214 is shown in view 230. The cursor 213 corresponds to the same location relative to the chromosome as cursor 233 in the zoomed view for perspective as to what is being shown. This view includes sufficient detail and space so that know transcripts 236 can be plotted alongside the other data in this view, in the locations that correspond to where they are found on the chromosome. This further aids the user's visual analysis, as the user may be familiar with one or more of the transcripts which is expected to be significantly altered, and when the visualization shows it appearing near one of the significant values of the Z-score plot 212, this serves as further confirmation/information to use in the analysis in an effort to explain the mechanisms that are occurring. Even if the microarrays that were used for the experiments do not have the transcripts annotated, the system can still identify the affected transcripts, since the genome is known.

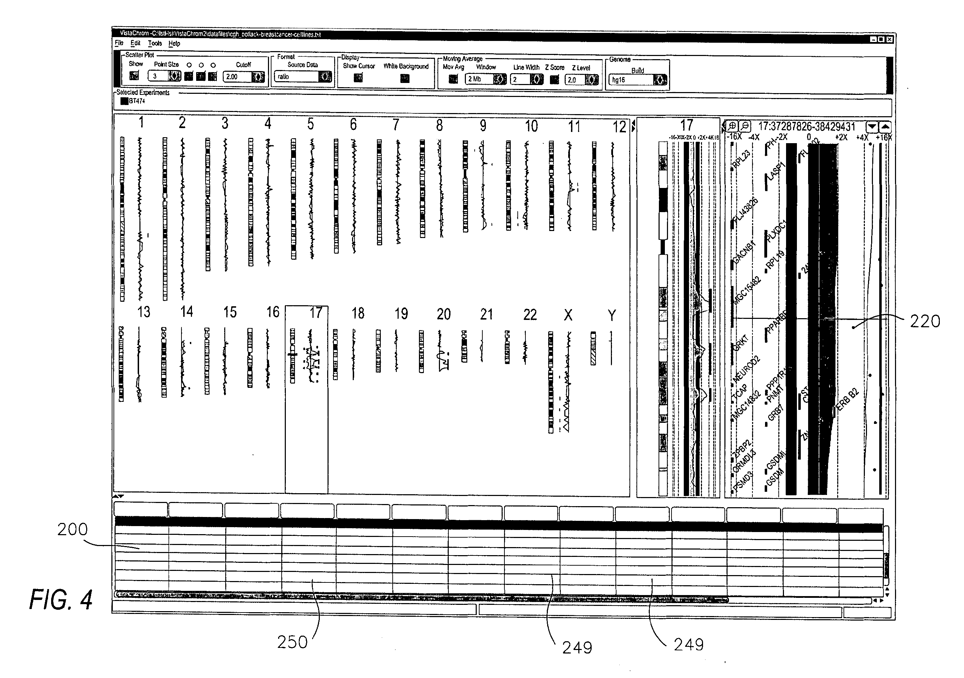

Further optionally, a scatter plot of all of the experimental data values 220 may be plotted in both the views 205 and 230, as shown in Fig. 4.

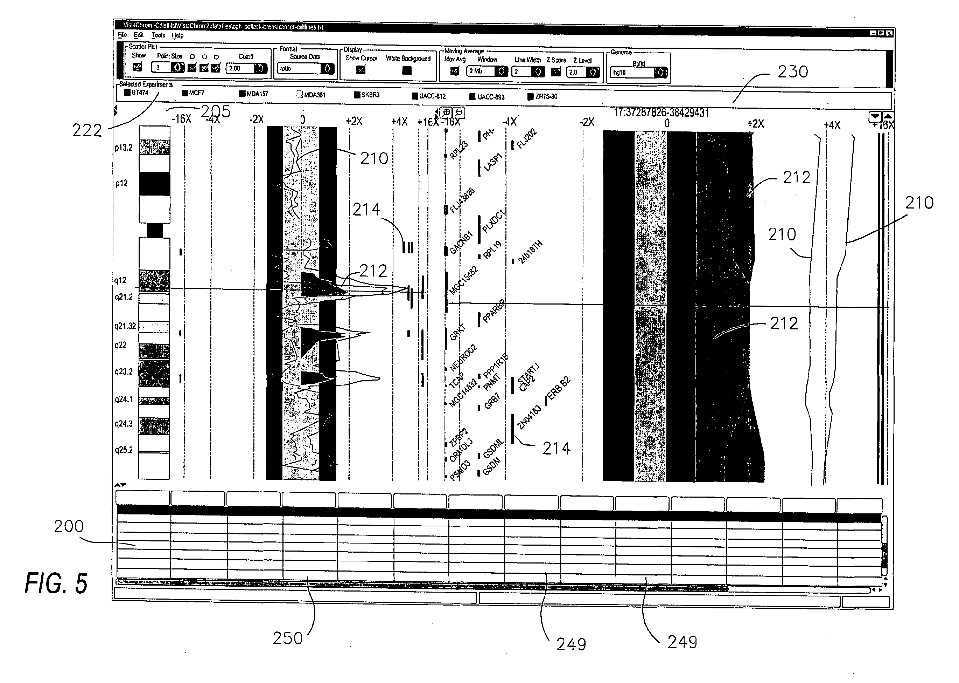

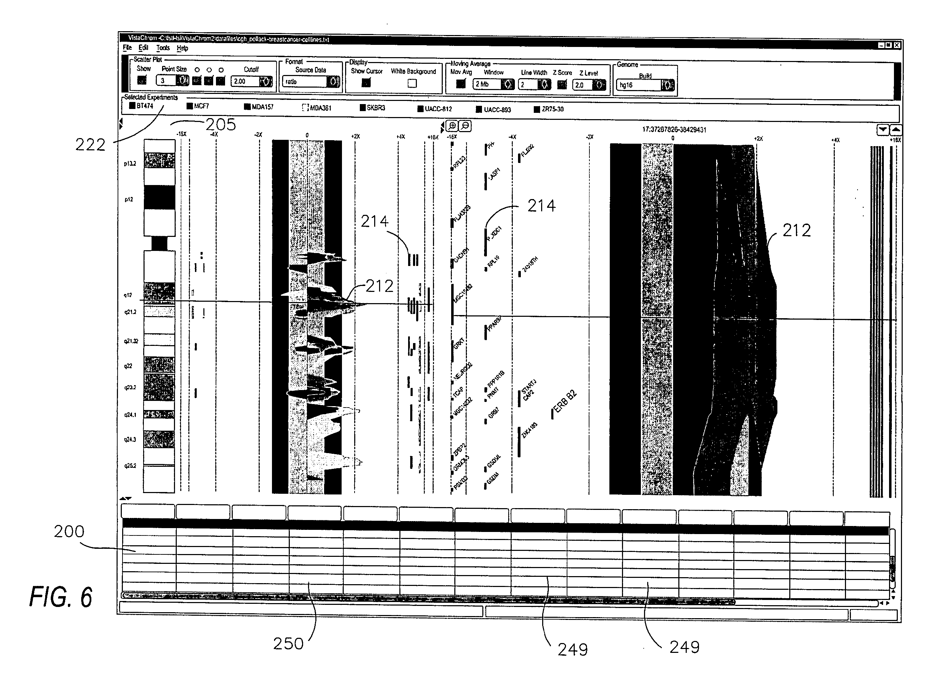

Fig. 5 shows a zoomed view of the portions 205 and 230 of the display of Fig. 3, where the global view of all chromosomes s not shown, so that the data, such as moving average data 210, Z-scores data 212 and sidebars 214 can be seen in greater detail. Fig. 6 shows a similar zoomed view, but shows data for eight experiments, as indicated in the "selected experiment" display 222. Moving average data has been selected not to be displayed, to provide clearer visualization of the z-scores data 212.

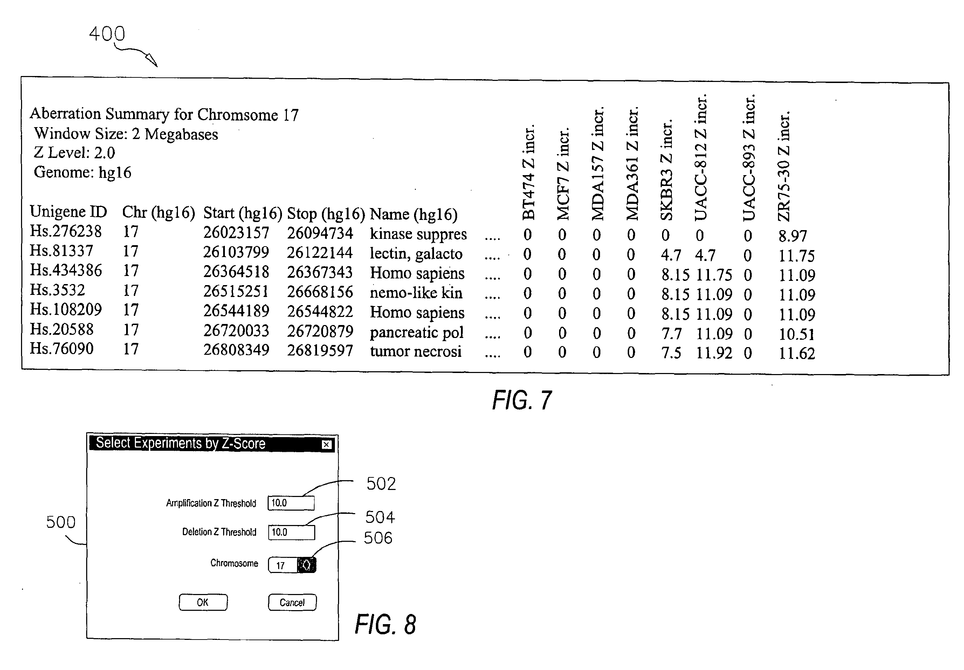

Further, the system provides a text reporter that outputs the raw data (e.g., array data adjacent Z-scores) in a spreadsheet type file, such as a Microsoft Excel® file, or the like. An exemplary portion 400 of such outputted raw data is shown in Fig. 7. Still further, the system may display an aberration summary in graphical form, such as in the form of a heat map, or other visual, graphic representation, for example. Co-pending, commonly owned Application Serial No. (Application Serial No,. not yet assigned, Attorney's Docket No. 10040244-2) filed on Sept. 29, 2004 and titled "Method and System for Analysis of Array-Based Comparative-Hybridization Data" describes further details regarding the graphical display of aberration data in an aberration summary. Application Serial No. (Application Serial No,. not yet assigned, Attorney's Docket No. 10040244-2) is hereby incorporated herein, in its entirety, by reference thereto.

As an alternative to simply selecting experimental data from which to plot the Z-scores plots 212 and, optionally, moving averages plots 210, such as by clicking on the columns in view 204, for example, the system also provides an interface 500 (see Fig. 8) in which the user can input an amplification Z-score threshold 502 and a deletion Z-score threshold 504 for selection of the experimental values with regard to the input of a selected chromosome at 506. In order for the data from a particular experiment to be displayed, at least one Z-score value for that experiment must exceed the inputted amplification Z-score threshold 502 or at least one Z-score value for that experiment must exceed the deletion Z-score threshold 504 that has been inputted. Once an experiment has "qualified", by meeting one of the criteria described, the entire dataset for that experiment is displayed.

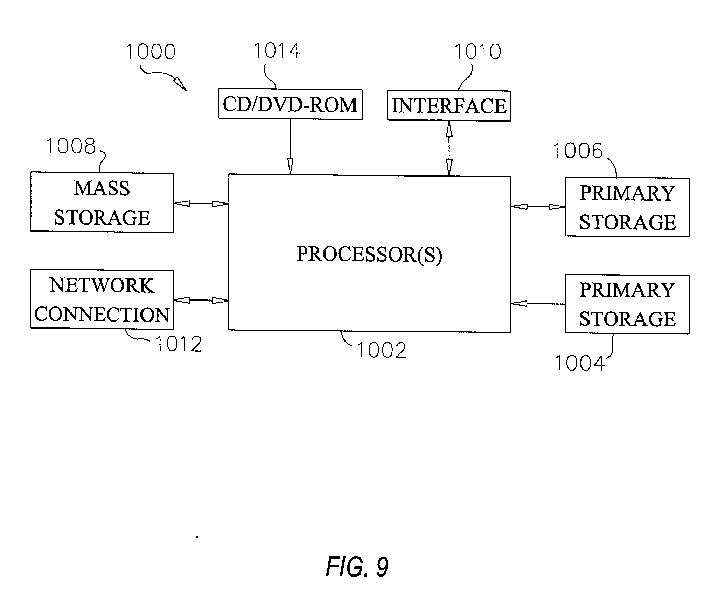

Fig. 9 illustrates a typical computer system in accordance with an embodiment of the present invention. The computer system 1000 includes any number of processors 1002 (also referred to as central processing units, or CPUs) that are coupled to storage devices including primary storage 1006 (typically a random access memory, or RAM), primary storage 1004 (typically a read only memory, or ROM). As is well known in the art, primary storage 1004 acts to transfer data and instructions uni-directionally to the CPU and primary storage 1006 is used typically to transfer data and instructions in a bi-directional manner Both of these primary storage devices may include any suitable computer-readable media such as those described above. A mass storage device 1008 is also coupled bidirectionally to CPU 1002 and provides additional data storage capacity and may include any of the computer-readable media described above. Mass storage device 1008 may be used to store programs, data and the like and is typically a secondary storage medium such as a hard disk that is slower than primary storage. It will be appreciated that the information retained within the mass storage device 1008, may, in appropriate cases, be incorporated in standard fashion as part of primary storage 1006 as virtual memory. A specific mass storage device such as a CD-ROM or DVD-ROM 1014 may also pass data uni-directionally to the CPU.

CPU 1002 is also coupled to an interface 1010 that includes one or more input/output devices such as such as video monitors, track balls, mice, keyboards, microphones, touch-sensitive displays, transducer card readers, magnetic or paper tape readers, tablets, styluses, voice or handwriting recognizers, or other well-known input devices such as, of course, other computers. Finally, CPU 1002 optionally may be coupled to a computer or telecommunications network using a network connection as shown generally at 1012. With such a network connection, it is contemplated that the CPU might receive information from the network, or might output information to the network in the course of performing the above-described method steps. The above-described devices and materials will be familiar to those of skill in the computer hardware and software arts.

The hardware elements described above may implement the instructions of multiple software modules for performing the operations of this invention. For example, instructions for calculating Z-scores may be stored on mass storage device 1008 or 1014 and executed on CPU 1008 in conjunction with primary memory 1006.

Methods of statistically analyzing apparent anomalies in CGH data may be implemented in hardware and/or software, wherein the CGH data includes a set of CGH ratio values ordered corresponding to locations of matter on chromosomes from which the CGH data was derived, and wherein the methods include the steps of: considering a subset of the set of CGH ratios defined by a window of predetermined size; computing a Z-test between the data within said window against statistics derived from said set of CGH ratios according to the following:

$$Z = \sqrt{n}\,\frac{\overline{X} - \mu}{\sigma} \qquad\qquad (4)$$

where $Z$ is the calculated value of the Z-test;
$n$ is the number of values within said window;
$\overline{X}$ is the mean of the values within said window;
$\mu$ is the mean of the values in said set; and
$\sigma$ is the standard deviation of the values in said set.; and calculating a moving average of the subset of values in the window.

Further, such a method may include moving the window of predetermined size by a predetermined incremental amount to define another subset of the set of CGH ratios and repeating said computing and calculating steps.

The moving and repeating steps may be repeated until all members of the set have been considered in at least one subset.

Methods may further include plotting the calculated values of the Z-tests and calculated values of the moving averages.

The plotting may include plotting the Z-test values and moving average values adjacent a chromosome map, in areas corresponding to the locations of the matter from which the CGH scores in the windows were derived, respectively. ,

The size of the window may be changed and then processing may be repeated to perform the computing and calculating steps noted above.

The CGH data may be aCGH data.

The CGH ratio values may be log ratios.

Methods of statistically analyzing apparent anomalies in CGH data may be implemented, wherein the CGH data includes a set of CGH ratio values ordered corresponding to locations of matter on chromosomes from which the CGH data was derived, including the steps of: considering a subset of the set of CGH ratios defined by a window of predetermined size; computing a t-test between the data within the window against statistics derived from the set of CGH ratios according to the following:

$$t = \sqrt{n}\,\frac{\overline{X} - \mu}{s}$$

where
$t$ is the calculated value of the t-test;
$n$ is the number of values within the window;
$\overline{X}$ is the mean of the values within the window;
$\mu$ is the mean of the values in the set; and
$s$ is the standard deviation of the values within the window.; and calculating a moving average of the subset of values in the window.

The window of predetermined size may be moved by a predetermined incremental amount to define another subset of the set of CGH ratios and then the computing and calculating steps may be repeated. ,

The moving and repeating steps may be repeated until all members of the set have been considered in at least one subset.

Further, the calculated values of the t-tests and calculated values of the moving averages may be plotted.

The plotting may include plotting the t-test values and moving average values adjacent a chromosome map, in areas corresponding to the locations of the matter from which the CGH scores in the windows were derived, respectively.

Further, the size of the window may be changed and then the computing and calculating steps may be repeated.

The CGH data may be aCGH data.

The CGH ratio values may be log ratios.

Methods are provided for statistically analyzing apparent anomalies in CGH data, wherein the CGH data is ordered corresponding to locations of matter on chromosomes from which the CGH data was derived, said method comprising the steps of: considering a set of CGH ratio values and computing a Z-normalized value for each CGH ratio value; classifying the Z-normalized values based upon a predetermined cutoff value; counting the number of Z-normalized values that are greater than the predetermined cutoff value, the number of Z-normalized values that are less than a negative of the predetermined cutoff value, and the total number of Z-normalized values; considering a subset of the set of CGH ratios defined by a window of predetermined size; and computing a Z-score to measure the significance of at least one of overabundance and underabundance of at least one of significant positive deviations and significant negative deviations in the subset.

Such methods may further include moving the window of predetermined size by a predetermined incremental amount to define another subset of the set of CGH ratios and repeating said computing step.

The moving and repeating steps may be repeated until all members of the set have been considered in at least one subset.

The methods may further comprise plotting at least one Z-score.

The plotting may include plotting at least one Z-score adjacent a chromosofne map, in an area corresponding to the location of the matter from which the CGH scores in the window were derived.

Further, a moving average of the subset of values in the window may be calculated. Such calculations may be performed for each subset incrementally defined by the window.

The Z-scores and moving averages may be plotted on the same display.

The Z-scores and moving averages may be plotted adjacent at least one chromosome map, in areas corresponding to the locations of the matter from which the CGH scores in the windows were derived, respectively.

The value of the predetermined cutoff value may be changed, and then the steps of classifying the Z-normalized values, counting the number of Z-normalized values, and computing a Z-score may be repeated, based upon the changed predetermined cutoff value.

Further, the size of the window may be changed and the steps of considering a subset of the set of CGH ratios may be repeated as defined by the changed size of the window, and from which a Z-score may be computed.

The CGH data may be aCGH data.

The CGH ratio values may be log ratios.

Each Z-normalized valued may be computed according to the following:

$$Z(x) = \frac{x - \mu}{\sigma} \qquad (1)$$

where
$Z(x)$ is said Z-normalized value;
x is the log of a measured CGH ratio;
$\mu$ is the mean of the log ratio values; and
$\sigma$ is the standard deviation of the population of the log ratio values in the set.

The Z-score may be computed by:

$$Z(w) = \frac{(r - n\frac{R}{N})}{\sqrt{n(\frac{R}{N})(1-\frac{R}{N})(1-\frac{n-1}{N-1})}} \qquad (2)$$

where
$Z(w)$ is the Z-score;
R is the number of counted Z-normalized values greater than the predetermined cutoff value;
N is the total number of said Z-normalized values;
r is the number of Z-normalized values within the window that are greater than the predetermined cutoff value; and
n is the total number of Z-normalized values within the window.

Further, the Z-score may then be computed by:

$$Z(w) = \frac{(r' - n\frac{R'}{N})}{\sqrt{n(\frac{R'}{N})(1 - \frac{R'}{N})(1 - \frac{n-1}{N-1})}} \qquad (3)$$

where

$Z(w)$ is the Z-score;

R' is said number of counted Z-normalized values less than the negative of the predetermined cutoff value;

N is the total number of Z-normalized values;

r' is the number of the Z-normalized values within the window that are less than the negative of the predetermined cutoff value; and

n is the total number of Z-normalized values within the window.

The methods may further include computing a Z-test between the data within the window against statistics derived from the set of data according to the following:

$$Z = \sqrt{n}\frac{\bar{X} - \mu}{\sigma} \qquad (4)$$

where

$Z$ is the calculated value of the Z-test;

$n$ is the number of values within the window;

$\bar{X}$ is the mean of the values within the window;

$\mu$ is the mean of the values in the set; and

$\sigma$ is the standard deviation of the values in the set.

The methods may further include computing a t-test between the data within said window against statistics derived from said set of data according to the following:

$$t = \sqrt{n}\frac{\bar{X} - \mu}{s}$$

where

$t$ is the calculated value of the t-test;

$n$ is the number of values within the window;

$\bar{X}$ is the mean of the values within the window;

$\mu$ is the mean of the values in the set; and

$s$ is the standard deviation of the values within the window.

The systems may further include means for moving the window of predetermined size by a predetermined incremental amount to define another subset of the set of CGH ratios and means for repeating said computing step.

The systems may further include means for plotting said Z-scores.

Further, the systems may include means for displaying a chromosome map, wherein the means for plotting plots the Z-scores adjacent the chromosome map, in areas corresponding to the locations of the matter from which the CGH scores in the windows were derived, for each respective Z-score.

Means for calculating a moving average of the subset of values in the window may be provided by the system.

The means for calculating may include means for calculating a moving average of each subset of values defined by each move of the window.

The systems may include means for plotting the Z-scores and moving averages.

The systems may further include means for displaying a chromosome map, wherein the means for plotting plots the Z-scores and the moving averages adjacent the chromosome map, in areas corresponding to the locations of the matter from which the CGH scores in the windows were derived, for each respective Z-score.

The systems may further include means for changing the value of the predetermined cutoff value and means for repeating the classification of Z-score values based upon the changed predetermined cutoff value, the counting the number of Z-scotes, and the computing a Z-score processes.

Further, the systems may include means for changing the size of the window and repeating the consideration of a subset of the set of CGH ratios defined by the changed size of the window, and the computing of a Z-score.

The CGH data processed by the systems may be aCGH data.

The systems may further include means for calculating CGH log ratios from the CGH ratio values. '

The systems may include means for computing a Z-test between the data within the window against statistics derived from the set of CGH data.

The systems may further include means for computing a t-test between the data within the window against statistics derived from the set of data.

Systems for statistically analyzing apparent anomalies in CGH data may be provided, wherein the CGH data includes a set of CGH ratio values ordered corresponding to locations of matter on chromosomes from which the CGH data was derived, a Z-score value has been computed for each CGH ratio value, the Z-scores have been classified based upon a predetermined cutoff value, and the number of Z-scores that are greater than the predetermined cutoff value, the number of Z-scores that are less than a negative of the predetermined cutoff value, and the total number of Z-scores have been counted; wherein a system includes: means for considering a subset of the set of CGH ratios defined by a window of predetermined size; and means for computing a Z-score to measure the significance of at least one of overabundance and underabundance of at least one of significant positive deviations and significant negative deviations in the subset.

Such a system may further include means for moving the window of predetermined size by a predetermined incremental amount along the set of CGH ratios to define another subset of the set of CGH ratios and means for repeating the computing of a Z-score.

The systems may further iterate the repeating and moving operations until all members of the set have been considered in at least one subset.

The systems may further include means for plotting the Z-score or scores.

The systems may include means for displaying a chromosome map, wherein the means for plotting plots the Z-scores adjacent the chromosome map, in areas corresponding to locations of the matter from which the CGH scores in the window were derived, with respect to each Z-score.

The systems may further include means for calculating a moving average of the subset of values in the window.

The systems may further include means for calculating a moving average of each subset of values defined by each move of the window.

The systems may further include means for plotting the Z-scores and the moving averages.

The systems may further include means for displaying a chromosome map, wherein the means for plotting plots the Z-scores and the moving averages adjacent the chromosome map, in areas corresponding to the locations of the matter from which the CGH scores in the windows were derived, with respect to each moving average and Z-score.

The systems may further include means for changing the size of the window and repeating the consideration of a subset of the set of CGH ratios defined by the changed size of the window, and the computing of a Z-score.

The CGH data processed by the systems may be aCGH data.

The systems may further include means for converting the CGH ratio values to CGH log ratio values.

The systems may further include means for displaying indicators adjacent plotted Z-scores having positive values that exceed the predetermined cutoff value and adjacent Z-scores having negative values that exceed a negative of the predetermined cutoff value.

The system may display sidebars as indicators.

The systems may further include means for displaying a zoomed view of the plotted Z-scores.

The systems may include means for displaying known transcripts in the zoomed view adjacent locations on the chromosome where they exist.

The systems may include means for displaying a graphical aberration summary.

The means for displaying a graphical aberration summary may display the graphical aberration summary in the form of a color-coded heat map.

The display of the graphical aberration summary and the display of the plotted Z-scores may be linked such that selecting an entry in one of the displays causes a cursor in the other of the displays to navigate to the same entry.

The systems may process aCGH data, and multiple arrays of aCGH data may be considered and processed to compute Z-scores and the systems may include means for plotting multiple plots of said Z-scores relating to the multiple arrays.

The systems may further include an interface for user selection of criteria for determining which of the multiple arrays to plot the Z-scores for.

In addition, embodiments of the present invention further relate to computer readable media or computer program products that include program instructions and/or data (including data structures) for performing various computer-implemented operations. The media and program instructions may be those specially designed and constructed for the purposes of the present invention, or they may be of the kind well known and available to those having skill in the computer software arts. Examples of computer-readable media include, but are not limited to, magnetic media such as hard disks, floppy disks, and magnetic tape; optical media such as CD-ROM, CDRW, DVD-ROM, or DVD-RW disks; magneto-optical media such as floptical disks; and hardware devices that are specially configured to store and perform program instructions, such as read-only memory devices (ROM) and random access memory (RAM). Examples of program instructions include both machine code, such as produced by a compiler, and files containing higher level code that may be executed by the computer using an interpreter.

Such computer readable media may carry one or more sequences of instructions for statistically analyzing apparent anomalies in CGH data, wherein the CGH data is ordered corresponding to locations of matter on chromosomes from which the CGH data was derived, wherein execution of one or more sequences of instructions by one or more processors causes the one or more processors to perform the steps of: considering a set of CGH ratio values and computing a Z-score value for each CGH ratio value; classifying the Z-score values based upon a predetermined cutoff value; counting the number of Z-scores that are greater than the predetermined cutoff value, the number of Z-scores that are less than a negative of the predetermined cutoff value, and the total number of Z-scores; considering a subset of the set of CGH ratios defined by a window of predetermined size; computing a Z-score to measure the significance of at least one of overabundance and underabundance of at least one of significant positive deviations and significant negative deviations in the subset.

Such computer readable media may carry one or more sequences of instructions for statistically analyzing apparent anomalies in CGH data, wherein the CGH data includes a set of CGH ratio values ordered corresponding to locations of matter on chromosomes from which the CGH data was derived, a Z-score value has been computed for each CGH ratio value, the Z-scores have been classified based upon a predetermined cutoff value, and the number of Z-scores that are greater than the predetermined cutoff value, the number of Z-scores that are less than a negative of the predetermined cutoff value, and the total number of Z-scores have been counted, wherein execution of one or more sequences of instructions by one or more processors causes the one or more processors to perform the steps of: considering a subset of the set of CGH ratios defined by a window of predetermined size; and computing a Z-score to measure the significance of at least one of overabundance and underabundance of at least one of significant positive deviations and significant negative deviations in the subset.

Thresholds for significance of Z-values may be somewhat subjectively set by the user. Typically, Z-values greater than three are considered significant, although some users consider Z-values greater than two to be significant. Thus, when choosing a value for $Z_c$ to classify Z-normalized values, a user might typically choose a value of two or three. However, most final Z-scores determined by the present systems and methods (i.e., the Z-scores that are calculated, not $Z_c$) are five to fifteen or higher, so that they almost always represent significant Z-scores. A Z-score of three corresponds to an approximate 95% confidence level that the value is not random. Thus, a Z-score of ten generally equates to a very high probability that the observed anomaly is not a random occurrence. However, in general, the Z-scores are not intended to be conclusive proof that the anomaly is real, but rather to show statistically where there are important anomalies. It is then up to the user to determine if the anomalies are appropriately statistically significant, and more importantly, whether such anomalies are biologically significant and relevant to the study at hand. The present methods are particularly interesting when an analysis is conducted with many experiments and where the results all agree on some statistically important anomaly. In such an instance, this is a strong indication of a shared anomaly that may be important in the mechanism of the disease being studied.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps. All such modifications are intended to be within the scope of the claims appended hereto.

**Claims**

1. A system for statistically analyzing apparent anomalies in CGH data, wherein a set of CGH data is ordered corresponding to locations of matter on chromosomes from which the CGH data was derived, said system comprising:

   means for inputting a set of CGH ratio values (102);
   means for computing a Z-normalized value for each CGH ratio value (106);
   means for classifying the Z-normalized values (108) based upon a predetermined cutoff value;
   means for counting the number of Z-normalized values that are greater than the predetermined cutoff value, the number of Z-normalized values that are less than a negative of the predetermined cutoff value, and the total number of Z-normalized values;
   means for considering a subset of the set of CGH ratios defined by a window of predetermined size (112); and

means for computing a Z-score (114) to measure the significance of at least one of overabundance and under-abundance of at least one of significant positive deviations and significant negative deviations in the subset.

2. The system of claim 1, further comprising means for moving the window of predetermined size by a predetermined incremental amount to define another subset of the set of CGH ratios and means for repeating said computing step.

3. The system of claim 1 or 2, further comprising means for calculating a moving average of the subset of values in the window.

4. The system of claim 2, further comprising means for calculating a moving average of each subset of values defined by each move of the window.

5. The system of any of claims 2-4, further comprising means for plotting said Z-scores (212).

6. The system of 5, further comprising means for displaying a chromosome map, wherein said means for plotting plots said Z-scores (212) adjacent the chromosome map (205), in areas corresponding to the locations of the matter from which said CGH scores in said windows were derived, for each respective Z-score.

7. The system of any of claims 3-6, further comprising means for plotting said moving averages (210).

8. The system of any of claims 1-7, further comprising means for changing the value of said predetermined cutoff value and means for repeating said classifying the Z-normalized values based upon the changed predetermined cutoff value, said counting the number of Z-normalized values, and said computing a Z-score.

9. The system of any of claims 1-8, further comprising means for changing the size of the window and repeating said considering a subset of the set of CGH ratios defined by the changed size of the window, and said computing a Z-score.

10. The system of any of claims 1-9, wherein the CGH data is aCGH data.

11. The system of any of claims 1-10, further comprising means for calculating CGH log ratios from said CGH ratio values.

12. The system of any of claims 1-11, further comprising means for computing a Z-test between the data within said window against statistics derived from said set of CGH data.

13. The system of any of claims 1-11, further comprising means for computing a t-test between the data within said window against statistics derived from said set of data.

14. The system of any of claims 1-13, further comprising means for displaying indicators adjacent plotted Z-scores having values that exceed said predetermined cutoff value.

15. The system of claim 14, wherein said indicators comprise sidebars (214).

16. The system of any of claims 1-15, further comprising means for displaying a zoomed view (230) of the Z-scores.

17. The system of claim 16, further comprising means for displaying known transcripts in said zoomed view (230) adjacent locations on the chromosome where they exist.

18. The system of any of claims 1-17, wherein said CGH data is aCGH data, wherein multiple arrays of aCGH data are considered and processed to compute Z-scores and wherein said system comprises means for plotting multiple plots of said Z-scores relating to said multiple arrays.

19. The system of claim 18, further comprising an interface for user selection of criteria for determining which of the multiple arrays to plot the Z-scores for.

20. The system of any of claims 1-18, including a user interface for displaying various graphical representations of CGH data values and apparent anomalies in the CGH data values, said user interface comprising:

means for displaying a chromosome map (205); and

means for plotting statistical scores of aberration characterizing the CGH data values adjacent the chromosome map, in areas corresponding to locations of matter from which said CGH data values were derived.

**21.** The system of claim 20, wherein said user interface further comprises

means for overlaying statistical scores (210,212) characterizing apparent anomalies in the CGH data values.

**22.** The system of claim 20 or 21, wherein said user interface displays the CGH data values as a scatter plot.

**23.** A method of statistically analyzing apparent anomalies in CGH data,

wherein the CGH data is ordered corresponding to locations of matter on chromosomes from which the CGH data was derived, said method comprising the steps of:

considering a set of CGH ratio values and computing a Z-normalized value (106) for each CGH ratio value;

classifying (108) the Z-normalized values based upon a predetermined cutoff value;

counting the number of Z-normalized values that are greater than the predetermined cutoff value, the number of Z-normalized values that are less than a negative of the predetermined cutoff value, and the total number of Z-normalized values;

considering a subset of the set of CGH ratios defined by a window of predetermined size;

computing a Z-score (114) to measure the significance of at least one of overabundance and underabundance of at least one of significant positive deviations and significant negative deviations in the subset. '

**24.** A method of statistically analyzing apparent anomalies in CGH data, wherein the CGH data includes a set of CGH ratio values ordered corresponding to locations of matter on chromosomes from which the CGH data was derived, a Z-score value has been computed for each CGH ratio value, the Z-scores have been classified based upon a predetermined cutoff value, and the number of Z-scores that are greater than the predetermined cutoff value, the number of Z-scores that are less than a negative of the predetermined cutoff value, and the total number of Z-scores have been counted; said method comprising the steps of:

considering a subset of the set of CGH ratios defined by a window of predetermined size; and

computing a secondary Z-score (114) to measure the significance of at least one of overabundance and underabundance of at least one of significant positive deviations and significant negative deviations in the subset.

FIG. 1

FIG. 2

FIG. 3

EP 1 647 911 A2

19

FIG. 4

FIG. 5

EP 1 647 911 A2

FIG. 6

400

Aberration Summary for Chromsome 17
Window Size: 2 Megabases
Z Level: 2.0
Genome: hg16

| Unigene ID | Chr (hg16) | Start (hg16) | Stop (hg16) | Name (hg16) | | BT474 Z incr. | MCF7 Z incr. | MDA157 Z incr. | MDA361 Z incr. | SKBR3 Z incr. | UACC-812 Z incr. | UACC-893 Z incr. | ZR75-30 Z incr. |
|---|---|---|---|---|---|---|---|---|---|---|---|---|---|
| Hs.276238 | 17 | 26023157 | 26094734 | kinase suppres | .... | 0 | 0 | 0 | 0 | 0 | 0 | 0 | 8.97 |
| Hs.81337 | 17 | 26103799 | 26122144 | lectin, galacto | .... | 0 | 0 | 0 | 0 | 4.7 | 4.7 | 0 | 11.75 |
| Hs.434386 | 17 | 26364518 | 26367343 | Homo sapiens | .... | 0 | 0 | 0 | 0 | 8.15 | 11.75 | 0 | 11.09 |
| Hs.3532 | 17 | 26515251 | 26668156 | nemo-like kin | .... | 0 | 0 | 0 | 0 | 8.15 | 11.09 | 0 | 11.09 |
| Hs.108209 | 17 | 26544189 | 26544822 | Homo sapiens | .... | 0 | 0 | 0 | 0 | 8.15 | 11.09 | 0 | 11.09 |
| Hs.20588 | 17 | 26720033 | 26720879 | pancreatic pol | .... | 0 | 0 | 0 | 0 | 7.7 | 11.09 | 0 | 10.51 |
| Hs.76090 | 17 | 26808349 | 26819597 | tumor necrosi | .... | 0 | 0 | 0 | 0 | 7.5 | 11.92 | 0 | 11.62 |

*FIG. 7*

Select Experiments by Z-Score [x]

500

Amplification Z Threshold [10.0] — 502

Deletion Z Threshold [10.0] — 504
— 506

Chromosome [ 17 ▼ ]

[ OK ]   [ Cancel ]

*FIG. 8*

EP 1 647 911 A2

1000   1014   1010

CD/DVD-ROM   INTERFACE

1008

MASS
STORAGE

1006

PRIMARY
STORAGE

PROCESSOR(S)

NETWORK
CONNECTION

1012

1002

PRIMARY
STORAGE

1004

FIG. 9